# EUROPEAN PATENT APPLICATION

(11) **EP 4 656 188 A1**
(43) Date of publication of application: **03.12.2025**
(21) Application number: 23918476.5
(22) Date of filing: 31.08.2023
(51) Int. Cl.: A61K 31/085, A61P 29/00, C12N 5/0786, C12Q 1/02

(54) **ANTI-INFLAMMATORY AGENT**

(30) Priority: 25.01.2023 JP 2023009164
(71) Applicant: Watanabe Oyster Laboratory Co., Ltd., Hachioji-Shi Tokyo 192-0154 (JP)
(72) Inventor: WATANABE Mitsugu, Hachioji-shi, Tokyo 192-0154 (JP)
(74) Representative: Plougmann Vingtoft a/s
(86) International application number: PCT/JP2023/031733
(87) International publication number: WO 2024/157516

(57) **Abstract**

[Problem]

The present invention demonstrates that, when added to the culture, DHMBA inhibits the proliferation of mouse inflammatory macrophage RAW264.7 cells, promotes their cell death, and reduces their cell number. It also demonstrates that DHMBA suppresses the increased production of inflammatory cytokines in RAW264.7 cells cultured with LPS, and in particular, DHMBA treatment suppresses osteoclast formation in RAW264.7 cells stimulated with LPS. Thus, the present invention provides a useful means of treating inflammatory diseases using DHMBA.

[Solution]

The present invention is characterized by having an anti-inflammatory effect by including 3,5-dihydroxy-4-methoxybenzyl alcohol (DHMBA) as an active ingredient.

## Description

### TECHNICAL FIELD

The present invention relates to an anti-inflammatory agent.

### BACKGROUND ART

Inflammation is involved in the pathogenesis of many diseases. Inflammatory cytokines are produced by macrophages in response to stimulation with lipopolysaccharide (LPS) and are used clinically as biomarkers involved in various pathological conditions.

A marine factor 3,5-dihydroxy-4-methoxybenzyl alcohol (hereinafter sometimes referred to as DHMBA) was discovered in the Pacific oyster (Crassostrea gigas).

DHMBA has the properties of reducing oxidative stress, scavenging radicals, and inducing antioxidant proteins in cells. However, the pharmacological role of DHMBA is poorly understood.

The present invention was performed to examine whether DHMBA suppressed proliferation, cytokine production, and osteoclast formation in inflammatory mouse macrophage RAW264.7 cells.

In cell culture using DHMBA (1 to 1000 µM), it was revealed that the proliferation of inflammatory murine macrophage RAW264.7 cells was suppressed in vitro, leading to cell death and a decrease in cell number. DHMBA was found to reduce the expression levels of signal transduction factors that promoted cell proliferation, such as Ras, PI3K, Akt, MAPK, phospho-MAPK, and mTOR, and to increase the expression levels of p53, p21, Rb, and regucalcin, which suppressed cell proliferation. Furthermore, DHMBA treatment increased the levels of caspase-3 and cleaved caspase-3, suggesting a mechanism for the induction of cell death.

LPS stimulation increased the production of inflammatory cytokines, such as tumor necrosis factor-α, interleukin-6, interleukin-1β, and prostaglandin E2. These increases were inhibited by culturing the cells with DHMBA. Notably, LPS treatment increased the level of NF-κB p65, and this increase was suppressed by DHMBA treatment. Further, in relation to this, LPS treatment promoted osteoclast formation in RAW264.7 cells, and this stimulation was blocked by DHMBA treatment. This has proved that DHMBA suppresses the activity of inflammatory macrophages in vitro, suggesting its therapeutic usefulness for inflammatory diseases.

### (Introduction)

Inflammation is a complex biological response of body tissues to injurious stimuli and is also a host-defense response in which immune cells, blood vessels, and molecular mediators cooperate. Inflammatory cytokines such as interleukins (ILs) and tumor necrosis factor (TNF)-α are known as biomarkers for chronic human muscle pain and osteoarthritis. These cytokines are produced by macrophages under inflammatory conditions. In particular, inflammatory macrophages have been emphasized for their potential to promote cancer cell progression, metastasis, and angiogenesis.

RAW264.7 cells are a monocyte/macrophage-like cell line. This cell line has been characterized with respect to macrophage-mediated immune, metabolic, and phagocytic functions. RAW264.7 cells are used as an in vitro model of macrophages under inflammatory conditions, and this cell line is noted as a model for osteoclast formation study. Osteoclasts are cells differentiated from the monocyte-macrophage lineage. Lipopolysaccharide (LPS) is a core antigen of Gram-negative bacteria and activates the host innate immune system. LPS is an endotoxin that provides a persistent inflammatory stimulus to tissues. LPS induces osteoclast formation in RAW264.7 cells by regulating NF-κB-related signaling pathways and transcriptional activity.

A novel phenolic antioxidant, 3,5-dihydroxy-4-methoxybenzyl alcohol (DHMBA), was originally identified from the Pacific oyster Crassostrea gigas. DHMBA has the properties of reducing oxidative stress, scavenging radicals, and inducing antioxidant proteins in cells. DHMBA has been shown to scavenge hydroxyl radicals. DHMBA has also been reported to have a preventive effect against excessive activity of glutamatergic neurons in rats and mice. DHMBA may be involved in regulating cellular functions as an antioxidant. Further, recent studies have demonstrated that DHMBA suppresses the proliferation of metastatic prostate cancer cells, providing a new strategy for the treatment of prostate cancer with DHMBA. Further, elucidating the pharmacological actions of DHMBA may be important in the treatment of various diseases.

The present invention has been conducted to determine whether DHMBA affects inflammatory macrophage activity in vitro and to assess its potential as a therapeutic agent for inflammatory diseases. As a result, in the present invention, it has been demonstrated that adding DHMBA to the culture inhibits the proliferation of mouse inflammatory macrophage RAW264.7 cells, promotes their cell death, and reduces their cell number.

Further, DHMBA has been found to suppress the increased production of inflammatory cytokines in RAW264.7 cells cultured with LPS. In particular, DHMBA treatment has been found to suppress osteoclast formation in RAW264.7 cells stimulated with LPS. Thus, the novel marine factor DHMBA may exert a pharmacological effect on inflammatory conditions caused by macrophages. The present invention provides a useful therapeutic means for inflammatory diseases using DHMBA.

### CITATION LIST

### PATENT LITERATURE

Patent Literature 1: JP 2017-132753 A

### DISCLOSURE OF THE INVENTION

### PROBLEMS TO BE SOLVED BY THE INVENTION

The present invention was conducted to determine whether DHMBA affected inflammatory macrophage activity in vitro and to assess its potential as a therapeutic agent for inflammatory diseases. As a result, in the present invention, it has been demonstrated that adding DHMBA to the culture inhibits the proliferation of mouse inflammatory macrophage RAW264.7 cells, promotes their cell death, and reduces their cell number.

Further, DHMBA has been found to suppress the increased production of inflammatory cytokines in RAW264.7 cells cultured with LPS. In particular, DHMBA treatment has been found to suppress osteoclast formation in RAW264.7 cells stimulated with LPS. Thus, the novel marine factor DHMBA may exert a pharmacological effect on inflammatory conditions caused by macrophages. The present invention provides a useful therapeutic means for inflammatory diseases using DHMBA.

### SOLUTIONS TO THE PROBLEMS

The present invention is characterized by:
having an anti-inflammatory effect by including 3,5-dihydroxy-4-methoxybenzyl alcohol (DHMBA) as an active ingredient; or
having an effect of suppressing activity of an inflammatory macrophage by including 3,5-dihydroxy-4-methoxybenzyl alcohol (DHMBA) as an active ingredient; or
having an effect of suppressing proliferation of a RAW264.7 cell by including 3,5-dihydroxy-4-methoxybenzyl alcohol (DHMBA) as an active ingredient; or
having an effect of suppressing proliferation of a RAW264.7 cell cultured with LPS by including 3,5-dihydroxy-4-methoxybenzyl alcohol (DHMBA) as an active ingredient; or
having an effect of promoting death of a RAW264.7 cell by including 3,5-dihydroxy-4-methoxybenzyl alcohol (DHMBA) as an active ingredient; or
having an effect of suppressing production of an inflammatory cytokine in a RAW264.7 cell by including 3,5-dihydroxy-4-methoxybenzyl alcohol (DHMBA) as an active ingredient; or
having an effect of suppressing production of an inflammatory cytokine in a RAW264.7 cell by including 3,5-dihydroxy-4-methoxybenzyl alcohol (DHMBA) as an active ingredient; or
having an effect of suppressing osteoclast formation in a RAW264.7 cell by including 3,5-dihydroxy-4-methoxybenzyl alcohol (DHMBA) as an active ingredient.

### EFFECTS OF THE INVENTION

The present invention was conducted to determine whether DHMBA affected inflammatory macrophage activity in vitro and to assess its potential as a therapeutic agent for inflammatory diseases. As a result, in the present invention, it has been demonstrated that adding DHMBA to the culture inhibits the proliferation of mouse inflammatory macrophage RAW264.7 cells, promotes their cell death, and reduces their cell number.

Further, DHMBA has been found to suppress the increased production of inflammatory cytokines in RAW264.7 cells cultured with LPS. In particular, DHMBA treatment has been found to suppress osteoclast formation in RAW264.7 cells stimulated with LPS. Thus, the novel marine factor DHMBA may exert a pharmacological effect on inflammatory conditions caused by macrophages. The present invention thus exhibits the effect of providing a useful therapeutic means for inflammatory diseases using DHMBA.

### BRIEF DESCRIPTION OF THE DRAWINGS

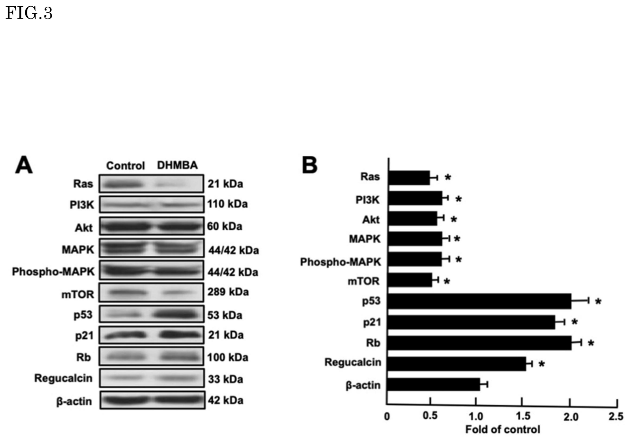

FIG. 1 is an explanatory diagram describing the chemical structure of 3,5-dihydroxy-4-methoxybenzyl alcohol (DHMBA). The molecular formula of DHMBA is C₈H₁₀O₄, and the molecular weight is 170.164.

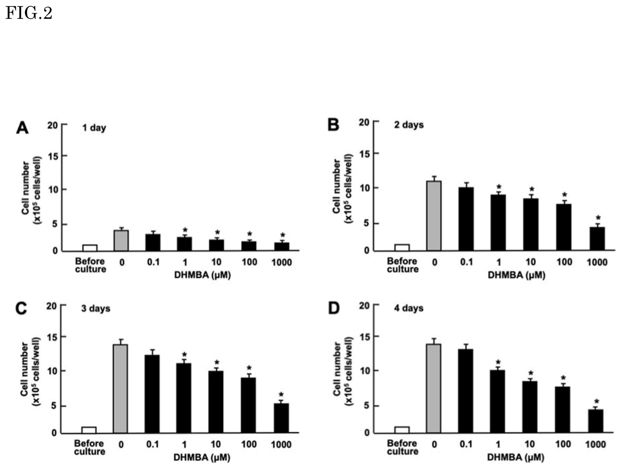

FIG. 2 is an explanatory diagram describing that the marine factor 3,5-dihydroxy-4-methoxybenzyl alcohol (DHMBA) suppresses the proliferation of mouse macrophage RAW264.7 cells. RAW264.7 cells (1×10⁵ cells/ml per well in a 24-well plate) were cultured in a DMEM culture medium containing 10% FBS (fetal bovine serum), 1% P/S (penicillin/streptomycin), and 1% fungizone in a control group (1% ethanol; 0 µM DHMBA as final concentration) and in a DHMBA-treated group (0.1, 1, 10, 100, or 1000 µM) for 1 day (panel A), 2 days (B), 3 days (C), or 4 days (D). After the culture, the number of cells attached to the dish was counted. Data are presented as the mean ± SD (standard deviation) from a total of 8 wells in 2 plates using different cell preparations. *: Significant difference (p<0.001) between the control group (gray bars) and the DHMBA-treated group. Statistical analysis was performed using 1-way ANOVA with Tukey-Kramer post-test.
FIG. 3 is an explanatory diagram describing that the marine factor 3,5-dihydroxy-4-methoxybenzyl alcohol (DHMBA) regulates the levels of various proteins related to the proliferation of mouse macrophage RAW264.7 cells.
   RAW264.7 cells (1×10⁶ cells/10 ml of medium in 100 mm dish) were cultured for 3 days in a DMEM culture medium containing 10% FBS, 1% P/S, and 1% fungizone in the control group (1% ethanol; 0 µM DHMBA as final concentration) and the DHMBA treated group (10 µM). After the culture, cells were removed from the dish using a cell scraper in a cell lysis buffer containing protease inhibitors. Forty micrograms of supernatant protein per lane were separated by electrophoresis on a 12% SDS-PAGE gel, transferred onto a nylon membrane, and probed by Western blotting with antibodies against various proteins. FIG. 3(A) shows representative data. In FIG. 3(B), band intensities are shown as fold increase over the control. Data are presented as the mean ± SD of values obtained from 4 dishes using different cell preparations. *: Significant difference (p<0.001) between the control group and the DHMBA-treated group. Statistical analysis was performed using 1-way ANOVA with Tukey-Kramer post-test.

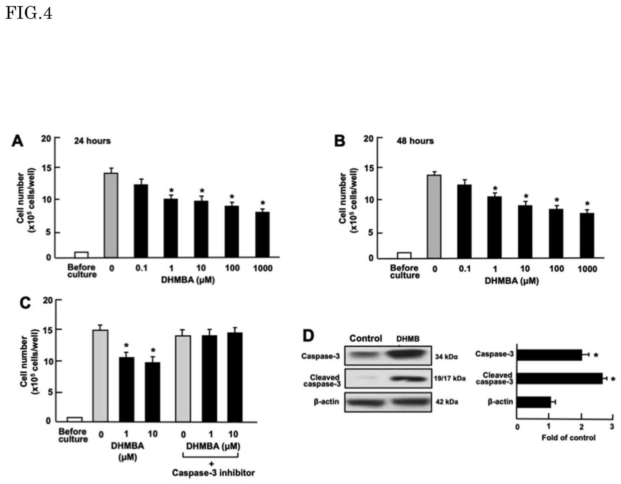

FIG. 4 is an explanatory diagram describing that the marine factor 3,5-dihydroxy-4-methoxybenzyl alcohol (DHMBA) stimulates cell death in mouse macrophage RAW264.7 cells. RAW264.7 cells (1×10⁵ cells/ml/well in a 24-well plate) were cultured in DMEM containing 10% FBS, 1% P/S, and 1% fungizone for 3 days until they reached subconfluence. The cells were cultured for another 24 hours (FIG. 4A) or 48 hours (FIG. 4B) in the control group (1% ethanol; 0 µM DHMBA as final concentration) and in the DHMBA-treated group (0.1, 1, 10, 100, or 1000 µM). In FIG. 4C, subconfluent RAW264.7 cells were cultured for another 48 hours with a caspase-3 inhibitor (10 µM) in the control group (1% ethanol; 0 µM DHMBA as final concentration) and the DHMBA-treated group (1 or 10 µM). After the culture, the number of cells attached to the dish was counted. Data are presented as the mean ± SD from a total of 8 wells in 2 plates using different cell preparations. In FIG. 4D, to measure the expression level of caspase-3 or cleaved caspase-3, PC-3 cells (1×10⁶ cells/10 ml medium in a 100 mm dish) were cultured for 3 days in a DMEM culture medium in the control group (1% ethanol; 0 µM DHMBA as final concentration) and the DHMBA-treated group (10 µM). After the culture, cell lysates were obtained for Western blotting assay. Representative data are shown in FIG. 4D. In FIG. 4E, band intensities are shown as fold increase over the control. Data are presented as the mean ± SD of values obtained from 4 dishes using different cell preparations. *: Significant difference (p<0.001) between the control group and the DHMBA-treated group. Statistical analysis was performed using 1-way ANOVA with Tukey-Kramer post-test.

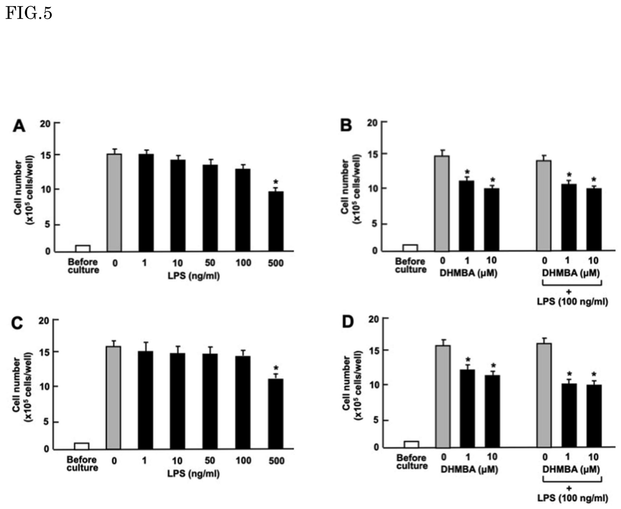

FIG. 5 is an explanatory diagram describing that the effects of 3,5-dihydroxy-4-methoxybenzyl alcohol (DHMBA) on the proliferation and death of mouse macrophage RAW264.7 cells are exhibited regardless of whether or not the cells are stimulated with lipopolysaccharide (LPS).
   In FIG. 5A, to examine the effect on cell proliferation, RAW264.7 cells (1×10⁵ /ml/well) were cultured for 3 days in a DMEM culture medium containing 10% FBS, 1% P/S, and 1% fungizone in the control group (1% ethanol as final concentration) with or without LPS (1, 10, 50, 100, 500 ng/ml). In FIG. 5B, cells (1×10⁵/ml per well) were cultured for 3 days in a DMEM culture medium containing 10% FBS, 1% P/S, and 1% fungizone in the DHMBA-treated group (1 or 10 µM) with or without LPS (100 ng/ml). In FIG. 5C, to examine the effect on cell death, cells that had reached subconfluence after 3 days of culture were cultured for another 48 hours in the control group (1% ethanol) with or without LPS (1, 10, 50, 100, or 500 ng/ml). In FIG. 5D, cells that had reached subconfluence were cultured for another 48 hours in the DHMBA-treated group (1 or 10 µM) with or without LPS (100 ng/ml). After the culture, the number of cells attached to the dish was counted. Data are presented as the mean ± SD from a total of 8 wells in 2 plates using different cell preparations. *: Significant difference (p < 0.01) versus the controls without LPS (gray bars) (FIG. 5A and FIG. 5C) or without DHMBA (gray bars) (FIG. 5B and FIG. 5D). Statistical analysis was performed by one-way ANOVA followed by the Tukey-Kramer post hoc test.

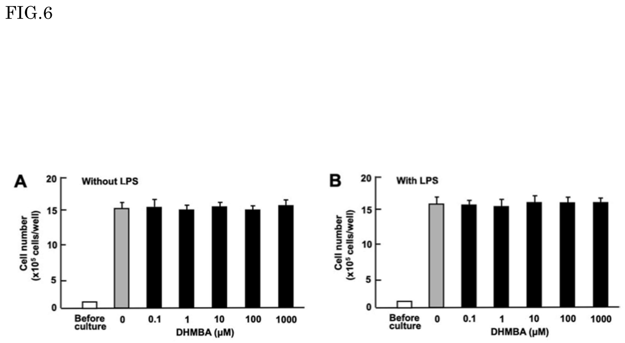

FIG. 6 is an explanatory diagram describing that 3,5-dihydroxy-4-methoxybenzyl alcohol (DHMBA) exerts its effect on the number of mouse macrophages RAW264.7 cells regardless of lipopolysaccharide (LPS) stimulation.
   RAW264.7 cells (1×10⁵/ml/well) were cultured in a DMEM culture medium containing 10% FBS, 1% P/S, and 1% fungizone. Cells were treated with a vehicle (1% ethanol; DHMBA 0 µM as final concentration) or DHMBA (0.1, 1, 10, 100, or 1000 µM) and incubated for 5 hours (FIG. 6A).
   Cells (1×10⁵/ml per well) were cultured for 3 days in a DMEM culture medium containing 10% FBS, 1% P/S, and 1% fungizone in the presence of LPS (100 ng/ml in all cultures). Cells were treated with a vehicle (1% ethanol; DHMBA 0 µM as final concentration) or DHMBA (0.1, 1, 10, 100, or 1000 µM) and incubated for 5 hours (FIG. 6B).
   After the culture, the number of cells attached to the dish was counted. Data are presented as the mean ± SD from a total of 8 wells of two plates using different cell preparations. Cell numbers were not significantly changed by DHMBA and/or LPS treatment compared to the controls (gray bars). No significant changes were observed by 1-way ANOVA with Tukey-Kramer post-test and/or LPS treatment compared to the controls (gray bars). Statistical analysis was performed using 1-way ANOVA with Tukey-Kramer post-test.

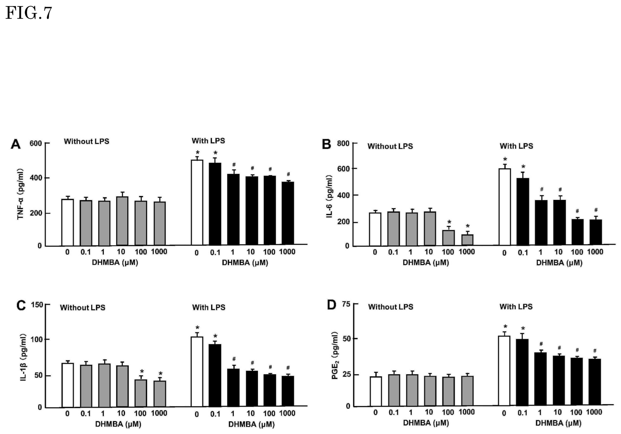

FIG. 7 is an explanatory diagram describing that the marine factor 3,5-dihydroxy-4-methoxybenzyl alcohol (DHMBA) suppresses the production of TNF-α, IL-6, IL-1β, and PGE₂ in mouse macrophage RAW264.7 cells stimulated with lipopolysaccharide (LPS).
   RAW264.7 cells (1×10⁵/ml per well in a 24-well plate) were cultured for 3 days in a DMEM culture medium without DHMBA until they reached subconfluence. After that, the cell culture was continued for another 5 hours in the control group (1% ethanol; DHMBA 0 µM as final concentration) and the DHMBA-treated group (0.1, 1, 10, 100, or 1000 µM) with or without LPS (100 ng/ml) in the cell culture medium. After the culture, the culture medium was collected, and the cytokine concentrations of TNF-α (FIG. 7A), IL-6 (FIG. 7B), IL-1β (FIG. 7C), and PGE₂ (FIG. 7D) in the culture medium were measured using an ELISA Kit. Data are presented as the mean ± SD from a total of 8 wells in two plates using different cell preparations. *: Significant difference (P<0.001) versus the controls without DHMBA and LPS (white bars). #: Significant difference (P<0.001) versus the controls with LPS but without DHMBA. Statistical analysis was performed using 1-way ANOVA with Tukey-Kramer post-test.

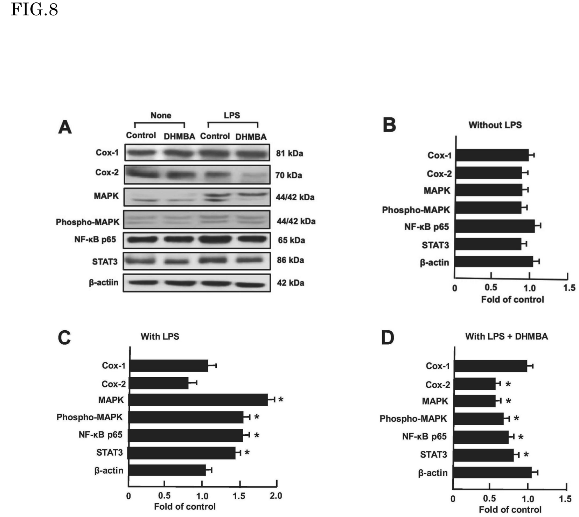

FIG. 8 is an explanatory diagram describing the effects of the marine factor 3,5-dihydroxy-4-methoxybenzyl alcohol (DHMBA) on the expression levels of COX-1, COX-2, MAPK, phosphorylated MAPK, NF-κB p65, and STAT3 in mouse macrophage RAW264.7 cells stimulated with lipopolysaccharide (LPS).
   Cells (1×10⁶ cells/10 ml of medium) were cultured in a DMEM culture medium for 3 days until they reached subconfluence. After that, cells were cultured for another 5 hours in the control group (1% ethanol; DHMBA 0 µM) and in the DHMBA-treated group (10 µM) with or without of LPS (100 ng/ml). After the culture, cell lysates were obtained for Western blot assay using specific antibodies against COX-1, COX-2, MAPK, phospho-MAPK, NF-κB p65, STAT3, and β-actin, as shown in FIG. 3.
   Representative data are shown in FIG. 8A. Band intensities are presented as fold increase over the controls, under the following conditions: no LPS (FIG. 8B), LPS (100 ng/ml) alone (FIG. 8C), and LPS (100 ng/ml) plus DHMBA (10 µM) (FIG. 8D). Data are presented as the mean ± SD of values obtained from 4 dishes using different cell preparations. *: Significant difference (p<0.01) versus the control without LPS (FIG. 8C). *: Significant difference (p<0.01) versus the control with LPS but without DHMBA (FIG. 8D). Statistical analysis was performed using 1-way ANOVA with Tukey-Kramer post-test.

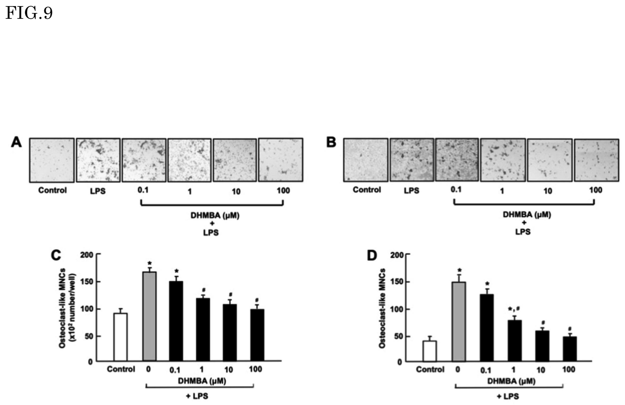

FIG. 9 is an explanatory diagram describing that the marine factor 3,5-dihydroxy-4-methoxybenzyl alcohol (DHMBA) inhibits osteoclast formation enhanced by lipopolysaccharide (LPS) stimulation in mouse macrophage RAW264.7 cells.
   Cells (1×10⁵ cells/1 ml per well in a 24-well plate) were cultured for 3 days in DMEM containing 10% FBS, 1% P/S, and 1% fungizone in the control group (1% ethanol as final concentration) and in the DHMBA-treated group (0.1, 1, 10, or 100 µM) with or without LPS (100 ng/ml). The old culture medium was replaced with fresh conditioned culture medium (0.5 ml) containing LPS and/or DHMBA, and the cells were cultured for another 3 days (FIG. 9A). Cells (1×10⁵ cells/1 ml per well in a 24-well plate) were cultured for 3 days in the above DMEM culture medium with LPS (100 ng/ml) but without DHMBA. Thereafter, 0.5 ml of the old medium was replaced with fresh medium (0.5 ml) containing LPS (100 ng/ml) and/or DHMBA (0.1, 1, 10, or 100 µM), and the cells were cultured for additional 3 days (FIG. 9B). After the culture, cells attached to the plate were fixed and stained for tartrate-resistant acid phosphatase (TRACP), a marker enzyme for osteoclasts. TRACP-positive multinucleated cells (MNCs) (with 3 or more nuclei) were counted as osteoclast-like cells under a microscope (40× magnification). Data are presented as the mean ± SD from a total of 8 wells in 2 plates using different cell preparations per data set. *: Significant difference (p<0.001) versus the control (white bar). #: Significant difference (p<0.001) versus LPS (gray bar). Statistical analysis was performed using 1-way ANOVA with Tukey-Kramer post-test.

### DESCRIPTION OF PREFERRED EMBODIMENTS

First, the present inventors cultured cells in the presence of DHMBA. As a result, it became clear that, using the cultured cells, DHMBA inhibited the proliferation of mouse inflammatory macrophage RAW264.7 cells, induced their cell death, and reduced their cell number.

Furthermore, DHMBA was found to suppress the increased production of inflammatory cytokines in RAW264.7 cells cultured with LPS. In particular, DHMBA treatment was found to suppress osteoclast formation in RAW264.7 cells stimulated with LPS. Thus, the novel marine factor DHMBA may exert a pharmacological effect on inflammatory conditions caused by macrophages. Thus, it has been confirmed that the present invention provides a useful therapeutic means for treating inflammatory diseases using DHMBA.

### (Test example)

### Materials and methods

### Reagents

Dulbecco's Modification of Eagle's Medium (DMEM) containing 4.5 g/L glucose, L-glutamine, and sodium pyruvate and antibiotics (100 units/mL penicillin and 100 µg/mL streptomycin; 1% P/S) were obtained from Corning Inc. (Mediach, Inc, Manassas, VA, USA).

Fetal bovine serum (FBS) was purchased from HyClone (Logan, UT, USA). Amphotericin B (fungizone), caspase-3 inhibitor (CAS 169332-60-9-Calbiochem), lipopolysaccharide (LPS), and all other reagents were purchased from Sigma-Aldrich (St. Louis, MO, USA) unless otherwise specified.

Caspase-3 inhibitor was diluted in sterile phosphate buffered saline (PBS). Other reagents were dissolved in 100% ethanol and stored at -20°C until use.

3,5-Dihydroxy-4-methoxybenzyl alcohol: 3,5-Dihydroxy-4-methoxybenzyl alcohol (DHMBA) is a novel amphiphilic phenolic compound that has been isolated, for example, from the Pacific oyster (Crassostrea gigas) as a compound with antioxidant properties. However, in the present invention, synthetic DHMBA is used. The synthetic DHMBA has the same effects as the DHMBA extracted from the Pacific oyster (Crassostrea gigas).

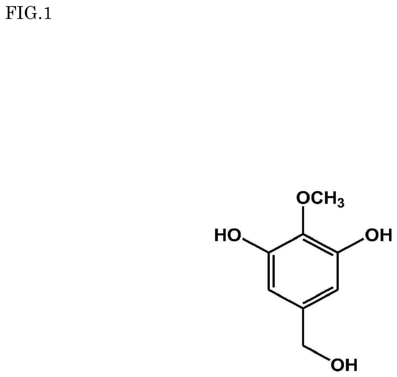

The chemical structure of DHMBA is shown in FIG. 1.

The purity of the synthetic DHMBA was 100%. The DHMBA was dissolved in, for example, 100% ethanol and stored at -20 °C until use.

### RAW264.7 cells

RAW264.7 cells are a monocyte/macrophage-like cell line, originating from Abelson leukemia virus transformed cell linage derived from BALB/c mice. Mouse macrophage RAW264.7 cells were obtained from the American Type Culture Collection (Rockville, MD, USA). RAW264.7 cells were cultured in Dulbecco's Modification of Eagle's Medium (DMEM) containing 10% FBS, 1% P/S, and 1% fungizone.

### Cell proliferation assay

RAW264.7 cells (1×10⁵/ml/well in a 24-well plate) were cultured for 3 days in a DMEM culture medium containing 10% FBS, 1% P/S, and 1% fungizone using a 24-well plate. Cells that had reached subconfluence were cultured for another 24 or 48 hours in a control group (PBS, or 1% ethanol; 0 µM DHMBA as final concentration) and a DHMBA-treated group (0.1, 1, 10, 100, or 1000 µM).

In another experiment, cells (1×10⁵/ml per well) were cultured for 3 days to subconfluence as described above and cultured for another 48 hours in the control group (1% ethanol; 0 µM DHMBA as final concentration) and the DHMBA-treated group (1 or 10 µM) with or without a caspase-3 inhibitor (10 µM).

In an additional experiment, to determine the effect of DHMBA on RAW264.7 cell death, RAW264.7 cells were cultured in the presence of LPS. These cells (1×10⁵/ml per well in a 24-well plate) were cultured for 3 days in a DMEM culture medium (10% FBS, 1% P/S, and 1% fungizone) until they reached subconfluence. The cells were then cultured for another 48 hours in the control group (1% ethanol as final concentration) and the LPS-treated group (1, 10, 50, 100, or 500 ng/ml in a culture medium) with or without DHMBA (1 or 10 µM). After the culture, the cells were detached with a Ca²⁺/Mg²⁺ free PBS solution with 0.05% trypsin/EDTA (0.1 ml per well) as described in the "Cell proliferation assay" section, and the cells were counted as described in the "Cell count" section below.

### Cell count

After the culture, to detach the cells from each well, a Ca²⁺/Mg²⁺ free PBS solution with 0.05% trypsin/EDTA was added to the culture dish (0.1 ml per well) and incubated at 37°C for 2 min. As described in the previous study, the cells were detached by adding DMEM (0.9 ml) containing 10% FBS and 1% P/S.

The number of viable cells was counted under a microscope (Olympus MTV-3) using a hemocytometer (Sigma-Aldrich) and a cell counter (H-102P, Line Seiki Co., Ltd., Tokyo, Japan).

For each dish, two separate counts were performed and averaged. Cell numbers are described as the count per well.

### Measurement of cytokine production

RAW264.7 cells (1×10⁵/ml per well) were cultured in a DMEM culture medium containing 10% FBS and 1% P/S using a 24-well plate for 3 days until they reached subconfluence. The cells were then cultured for another 5 hours in the control group (1% ethanol; DHMBA 0 µM as final concentration) and in the DHMBA-treated group (0.1, 1, 10, 100, or 1000 µM) with or without LPS (100 ng/ml). After the culture, the culture media were collected to measure cytokines. Then, the cells were detached from each culture dish to measure the cell number by the method described in "Cell count". The concentrations of TNF-α, IL-6, IL-1β, and PGE₂ in the culture medium were measured using ELISA kits. For the measurement, kits for TNF-α (cat. no. BM), KHC301) and IL-1β (cat. no. BMS6002) were obtained from ThermoFisher Scientific (Waltham, MA, USA), and kits for IL-6 (cat. no. 583371) and PGE₂ (cat. no. 514010) were purchased from Cayman Chemical (Ann Arbor, MI, USA). The amount of each cytokine produced was expressed as pictograms (pg) secreted into the culture medium (ml).

To measure the expression levels of Cox-1, Cox-2, NF-κB p65, and STAT3, which are involved in cytokine signaling, RAW264.7 cells (1×10⁶ cells/10 ml of 100 mm dish) were cultured in a DMEM culture medium containing 10% FS, 1% P/S, and 1% fungizone for 3 days until they reached subconfluence, and then cultured for another 5 hours in the control group (1% ethanol; DHMBA 0 µM as final concentration) and in the DHMBA-treated group (10 µM) with or without LPS (100 ng/ml in the medium). After the culture, the cells attached to the dish were scraped off with a cell lysis buffer and collected for measurement as described in the "Western blotting" section.

### Western blotting

RAW264.7 cells (1×10⁶ cells/10 ml of 100 mm dish) were cultured for 3 days in 10% FBS, 1% P/S (1% ethanol), or in the DHMBA-treated group (10 µM). After the culture, the dishes were washed with cold PBS (10 ml) to remove floating and dead cells. The cell lysis buffer (Cell Signaling Technology, Inc., Danvers, MA, USA) supplemented with protease/protein phosphatase inhibitor (Roche Diagnostics, Indianapolis, IN, USA) was added to the cells to collect cell lysates of the adherent cells as described in the previous study. The cell lysates were centrifuged at 17,000×g, 4°C for 10 min. Protein concentrations in the supernatants were measured using Bio-Rad Protein Assay Dye (Bio-Rad Laboratories, Inc., Hercules, CA, USA) with bovine serum albumin as a standard. The cell lysates were stored at -80°C until use. The supernatant proteins (40 µg/lane) were separated by SDS-polyacrylamide gel electrophoresis (12% SDS-PAGE) and transferred to a PVDF membrane. The transferred membranes were immunoblotted with specific antibodies against various proteins, including Ras (cat. no. 3339, rabbit), Akt (cat. no. 9272, rabbit), mitogen-activated protein kinase (MAPK; cat. no. 4695, rabbit), phosphorylated MAPK (cat. no. 4370, rabbit), mechanistic target of rapamycin (mTOR; cat. no. 4517, mouse), Rb (cat. no. 9309, mouse), p21 (cat. no. 2947, rabbit), STAT3 (cat. no. 12640, rabbit), COX-1 (cat. no. 48415), COX-2 (cat. no. 4842), and β-actin (cat. no. 3700, mouse), all of which were purchased from Cell Signaling Technology, Inc. (Danvers, MA, USA). Specific antibodies for p53 (cat. no. sc-126, mouse) and NF-κB p65 (cat. no. sc-109, rabbit) were purchased from Santa Cruz Biotechnology Inc. (Santa Cruz, CA, USA). Additionally, rabbit anti-regucalcin antibody (cat. no. HPA029103, rabbit) was obtained from Sigma-Aldrich. Target proteins were incubated overnight at 4°C with one of the above primary antibodies (1:1,000). After incubation, the membranes were further incubated with horseradish peroxidase-conjugated secondary antibodies (Santa Cruz Biotechnology Inc., cat. no. sc-2005 (mouse) or sc-2305 (rabbit); dilution 1:2000) for 60 min at room temperature, and protein bands were detected using chemiluminescence substrates (cat. no. 34577, Thermo Fisher Scientific Inc., Rockford, IL, USA) on X-ray films. From 4 independent experiments, a total of 3 or 4 films with separate membranes were scanned with Epson Perfection 1660 Photo scanner to quantify the bands using Image J2 software (National Institutes of Health, Bethesda, MD, USA). In addition, for immunoblotting using additional antibodies, the membranes were incubated at room temperature for 30 min with Restore (registered trademark) Western Blot Stripping Buffer (cat. no. 21059; Thermo Fisher Scientific Inc., Rockford, IL, USA) to remove attached chemiluminescence substrates (Thermo Fisher Scientific Inc.,) and were used to measure other protein molecules.

### Measurement of osteoclast formation

RAW264.7 cells (1×10⁵ cells/1 ml per well in a 24-well plate) were cultured for 3 days in a DMEM culture medium containing 10% FBS, 1% P/S, and 1% fungizone in the control group (1% ethanol in the culture medium as final concentration) and the LPS-treated group (100 ng/ml in the culture medium) with or without DHMBA (0.1, 1, 10, or 100 µM). The old medium was replaced with fresh medium (0.5 ml) containing LPS or DHMBA described above, and the cells were cultured for another 3 days. In another experiment, RAW264.7 cells (1×10⁵ cells/1 ml per well in a 24-well plate) were cultured for 3 days in DMEM containing 10% FBS, 1% P/S, and 1% fungizone in the control group (1% ethanol in the culture medium as final concentration) and the LPS-treated group (100 ng/ml in the culture medium) without DHMBA. Then, the old medium (0.5 ml) was replaced with fresh medium (0.5 ml) including DHMBA (0.1, 1, 10, or 100 µM in the final concentration) in the control group (1% ethanol as final concentration) and the LPS-treated group (100 ng/ml in the medium), and the cells were cultured for 3 days. RAW264.7 cells attached to the plate were fixed and stained for tartrate-resistant acid phosphatase (TRACP), a marker enzyme for osteoclasts. After the culture, the cells were washed with PBS solution and fixed with 10% neutralized formalin-phosphate (pH 7.2) for 1 min for staining. The fixed cells were incubated at room temperature for 10 hours in an acetate buffer (pH 5.0) containing naphthol AS-MX phosphate (Sigma-Aldrich) as a stain for reaction product in the presence of 10 mM sodium tartrate. TRACP-positive multinucleated cells (MNCs) having three or more nuclei were counted as osteoclast-like cells under a microscope (40× magnification) (Olympus MTV-3; Olympus Corp., Tokyo, Japan). To evaluate the number of osteoclast-like TRACP-positive MNCs, one field per well was imaged and the cells were measured using ImageJ2 software. The number of osteoclast-like TRACP-positive MNCs was then counted in five randomly selected fields under a light microscope (40×) (Olympus MTV-3; Olympus Corp., Tokyo, Japan), and the average was calculated.

### Statistical analysis

Statistical significance was estimated using GraphPad InStat version 3 for Windows XP (GraphPad Software Inc. La Jolla, CA). Data are presented as the mean ± standard deviation (SD). Student-t-test was used to calculate statistical significance between two groups. Multiple comparisons were performed by one-way analysis of variance (ANOVA) with Tukey-Kramer multiple comparison post-test for parametric data as indicated. A p-value of less than 0.05 was considered statistically significant.

### Results

DHMBA suppresses the proliferation of RAW264.7 cells.

First, it was investigated whether DHMBA affected the proliferation of mouse macrophage RAW264.7 cells (FIG. 2). RAW264.7 cells were cultured for 1, 2, 3, or 4 days in a control group (1% ethanol; DHMBA 0 µM as final concentration) and in a DHMBA-treated group (0.1, 1, 10, 100, or 1000 µM). The proliferation of RAW264.7 cells was inhibited by culturing them with DHMBA (1, 10, 100, or 1000 µM) for 1 to 4 days. Thus, DHMBA was found to suppress the proliferation of RAW264.7 cells in vitro.

To better understand the underlying mechanism by which DHMBA suppresses the proliferation of RAW264.7 cells, it was examined whether DHMBA regulated the expression of key proteins related to the proliferation of RAW264.7 cells (FIG. 3). It was found that incubation of cells with DHMBA (10 µM) decreased the levels of Ras, PI3 kinase, Akt, MAPK, phospho-MAPK, and mTOR, which were involved in promoting the proliferation of RAW264.7 cells, and increased the expression level of regucalcin, which led to the suppression of cell proliferation. These results suggest that the mechanism by which DHMBA suppresses the proliferation of RAW264.7 cells involves changes in signal transduction proteins and cell proliferation inhibitors.

### DHMBA promotes RAW264.7 cell death.

It was further examined whether DHMBA affected cell death of mouse macrophage RAW264.7 cells. Cells were cultured for 3 days to subconfluence and then cultured for another 24 or 48 hours in the control group (1% ethanol; DHMBA 0 µM as final concentration) and in the DHMBA-treated group (0.1, 1, 10, 100, or 1000 µM). Death in RAW264.7 cells was promoted by culturing them with DHMBA (1, 10, 100, or 1000 µM) for 24 hours (FIG. 4A) or 48 hours (FIG. 4B). The stimulatory effect of DHMBA (1 or 10 µM) on the death of RAW264.7 cells was blocked by the presence of a caspase-3 inhibitor (10 µM) (FIG. 4C). Western blotting results showed that incubation with DHMBA (10 µM) increased the levels of caspase-3 and cleaved caspase-3 in the cells (FIG. 4D). These results suggest that DHMBA stimulates apoptotic cell death in mouse macrophage RAW264.7 cells.

### Effect of DHMBA on RAW264.7 cells cultured with LPS

Next, the inhibitory effect of DHMBA on inflammatory macrophage RAW264.7 cells treated with LPS was investigated. It is well known that LPS enhances the inflammatory activity of mouse macrophage RAW264.7 cells. It was investigated whether DHMBA attenuated the proliferation suppressing or death promoting effects in vitro on RAW264.7 cells cultured in the presence of LPS (FIG. 5). RAW264.7 cells were cultured for 3 days in the presence of LPS (1, 10, 50, 100, or 500 ng/ml in the culture medium). Although culturing with LPS (1, 10, 50, or 100 ng/ml) did not affect the proliferation of RAW264.7 cells, culturing with a higher concentration of LPS (500 ng/ml) suppressed the proliferation (FIG. 5A). In the presence of LPS (100 ng/ml), the proliferation of RAW264.7 cells was also suppressed by DHMBA (1 or 10 µM) (FIG. 5B).

Thus, the suppressing effect of DHMBA on the cell proliferation was exhibited even in the presence of LPS.

To examine the effect of DHMBA on cell death in cells cultured in the presence of LPS, RAW264.7 cells that had been cultured for three days to reach subconfluence were cultured for another 48 hours in the culture medium containing LPS (1, 10, 50, 100, or 500 ng/ml) (FIG. 5C). Incubation with LPS (1, 10, 50, or 100 ng/ml) did not significantly affect the cell number, but the addition of a higher concentration of LPS (500 ng/ml) promoted the death of RAW264.7 cells. In the presence of LPS (100 ng/ml), the effect of DHMBA (1 or 10 µM) on RAW264.7 cell death was exhibited (FIG. 5D). These results indicated that DHMBA retained the activity of reducing the number of RAW264.7 cells in the presence of LPS (100 ng/ml).

### DHMBA suppresses inflammatory cytokine production in RAW264.7 cells.

It was further investigated whether DHMBA affected inflammatory cytokine production in vitro in mouse macrophage RAW264.7 cells cultured in the presence of LPS (100 ng/ml).

RAW264.7 cells were cultured for 3 days to subconfluence, then, after addition of LPS (100 ng/ml), incubated for 5 hours with or without DHMBA (0.1, 1, 10, 100, 1000 µM). The number of RAW264.7 cells remained unchanged in the absence of LPS (FIG. 6A) and in the presence of LPS (100 ng/ml in the culture medium) (FIG. 6B).

The production of major inflammatory cytokines, including TNF-α (FIG. 7A), IL-6 (FIG. 7B), IL-1β (FIG. 7C), and PGE₂ (FIG. 7D), was measured in the culture medium obtained by culturing RAW264.7 cells under the same culture conditions that did not affect the cell numbers. In the absence of LPS, the production of IL-6 and IL-1β in RAW264.7 cells was suppressed by the addition of DHMBA (100 or 1000 µM) (FIGS. 7B and C). Notably, LPS treatment significantly increased the production of TNF-α (FIG. 7A), IL-6 (FIG. 7B), IL-1β (FIG. 7C), and PGE₂ (FIG. 7D). These increases were suppressed by the addition of DHMBA (1, 10, 100, or 1000 µM). Thus, it was found that the production of inflammatory cytokines in RAW264.7 cells was suppressed by DHMBA.

It was investigated whether DHMBA was involved in cytokine production and regulated the expression levels of proteins related to intracellular cytokine signal transduction processes (FIG. 8).

RAW264.7 cells were incubated in vitro for 5 hours in the presence of LPS (100 ng/ml) with or without DHMBA (10 µM). In RAW264.7 cells cultured without LPS, the levels of COX-1, COX-2, MAPK, phospho-MAPK, NF-κB p65, and STAT3 were not altered by treatment with DHMBA compared to the control (FIGS. 8A and 8B). The levels of MAPK, phospho-MAPK, NF-κB p65, and STAT3 in RAW264.7 cells were increased by culturing the cells with LPS (100 ng/ml) (FIGS. 8A and 8C). These increases were suppressed by DHMBA treatment (10 µM) (FIGS. 8A and 8D). These results suggest that DHMBA treatment suppresses the levels of signal transduction factors involved in cytokine production in inflammatory RAW264.7 cells.

DHMBA suppresses osteoclast formation in RAW264.7 cells.

RAW264.7 cells are often used as a model for studying osteoclast formation. Osteoclasts are cells differentiated from monocyte-macrophage lineage. LPS has been shown to stimulate osteoclast formation in RAW264.7 cells by activating the NF-κB signaling pathway. It was therefore examined whether DHMBA affected osteoclast formation in RAW264.7 cells cultured with LPS. RAW264.7 cells (1×10⁵ cells/1 ml per well in a 24-well plate) were cultured for 3 days in a DMEM culture medium containing 10% FBS, 1% P/S, and 1% fungizone. After 3 days of culture in the control group (1% ethanol as final concentration) and the LPS-treated group (100 ng/ml in the culture medium) with or without DHMBA (0.1, 1, 10, or 100 µM), the old medium (0.5 ml) was replaced with fresh medium (0.5 ml) containing LPS (100 ng/ml) or DHMBA (0.1, 1, 10, or 100 µM), and the cells were cultured for another 3 days. When the cells were cultured for 6 days in the presence of both LPS and DHMBA (FIGS. 9A and 9C), LPS enhanced osteoclast formation in RAW264.7 cells. This enhancement was suppressed by the addition of DHMBA (0.1, 1, 10, or 100 µM) to the culture (FIGS. 9A and 9C).

In another experiment, RAW267.4 cells were cultured for 3 days in the presence of LPS (100 ng/ml) but without DHMBA. Then, the medium was replaced with fresh medium containing LPS (100 ng/ml), and the cells were cultured for another 3 days with the addition of DHMBA (0.1, 1, 10, or 100 µM). DHMBA exerted the suppressing effect even at the late stage of osteoclast formation enhanced by LPS (FIGS. 9B and 9D). These results supported that DHMBA suppressed LPS-stimulated osteoclast formation in RAW264.7 cells (FIGS. 9B and 9D).

### Discussion

The novel phenolic antioxidant, 3,5-dihydroxy-4-methoxybenzyl alcohol (DHMBA), has two properties: it scavenges radicals and attenuates oxidative stress in cells. DHMBA may be involved in regulating cell functions as an antioxidant. In the previous study, the present inventors showed that DHMBA suppressed the growth and activity of metastatic prostate cancer cells by targeting various signal transduction pathways, providing a new strategy for prostate cancer treatment. The present inventors further investigated whether DHMBA exhibited anti-inflammatory effects in vitro using mouse macrophage RAW264.7 cells. As a result, it was found that DHMBA exhibited anti-inflammatory effects in RAW264.7 cells, such as reducing the cell number of inflammatory macrophages, suppressing the production of inflammatory cytokines, and suppressing osteoclast formation. DHMBA was confirmed to be useful in the treatment of inflammatory diseases.

Incubation with DHMBA suppressed the proliferation of RAW264.7 cells, promoted cell death, and reduced the number of macrophages. DHMBA reduced the expression levels of Ras, PI3 kinase, Akt, MAPK, phospho-MAPK, and mTOR, which were known to promote the proliferation of RAW264.7 cells, and increased the expression levels of p53, Rb, and p21, which were known to suppress the cell proliferation. The reduction in the levels of these cell signaling-related proteins and the increase in the cell proliferation inhibitors by DHMBA may contribute to the fundamental mechanisms by which this compound inhibits the proliferation of RAW264.7 cells.

This suggests that DHMBA may regulate the expression of various proteins related to cell signal transduction and transcriptional activity. Furthermore, the levels of caspase-3 and cleaved caspase-3, which are involved in apoptotic cell death, were increased by DHMBA treatment. These enhancements may lead to the activation of nuclear DNA fragmentation, which induces apoptotic cell death. Thus, DHMBA may affect the levels of various proteins related to the control of cell number.

In addition, DHMBA was found to strongly suppress the production of inflammatory cytokines such as TNF-α, IL-6, IL-1β, and PGE₂ in macrophage RAW264.7 cells treated with LPS, which stimulated the inflammatory activity of the cells. These decreases were observed under culture conditions in which the number of RAW264.7 cells did not decrease even when the cells were cultured with both LPS and DHMBA. It was also found that DHMBA suppressed the cytokine production regardless of the change in the number of RAW264.7 cells. It has been shown that LPS treatment enhances the production of TNF-α, IL-6, IL-1β, or PGE₂ in RAW264.7 cells.

The present inventors found that the production of these cytokines, which was enhanced by LPS treatment, was suppressed in vitro by culturing RAW264.7 cells with DHMBA. These findings suggest that DHMBA treatment may serve as a useful tool for suppressing cytokine production under inflammatory conditions.

Further studies were performed to understand the fundamental mechanisms by which DHMBA suppressed the cytokine production in LPS-stimulated inflammatory RAW264.7 cells. LPS binds to Toll-like receptor 4 (TLR4) on the cell membranes of macrophage RAW264.7 cells, and the LPS/TLR4 pathway signaling is transmitted into the cells. In particular, TLR4 signaling activates NF-κB and MAPK signaling in RAW264.7 cells. In addition, evidence has been accumulated that LPS binds to intracellular proteins and receptors. The production of TNF-α, IL-6, IL-1β, and PGE₂ by LPS treatment may be involved in intracellular signal transduction of NF-xB p65 and MAPK in RAW264.7 cells. COX-1 and COX-2 are involved in the production of PGE₂ in RAW264.7 cells. It was found that incubation of RAW264.7 cells with LPS increased the levels of NF-κB p65, MAPK, and phosphorylated MAPK. These increases were suppressed by DHMBA treatment. This suppression may be related to the suppression of LPS-induced inflammatory cytokine production in RAW264.7 cells. Furthermore, DHMBA may inhibit the binding of LPS to TLR4 or intracellular receptor proteins, thereby attenuating the production of inflammatory cytokines in RAW264.7 cells. It is also speculated that DHMBA regulates the transcriptional activity related to the production of inflammatory cytokines in RAW264.7 cells. Furthermore, DHMBA may also be involved in the suppression of cytokine production as an antioxidant.

Interestingly, the level of STAT3 was increased by LPS treatment, and this increase was suppressed by culturing RAW264.7 cells with DHMBA. STAT3 is involved in the intracellular signal transduction of IL-6. LPS stimulation induced the production of IL-6 and IL-1β in RAW264.7 cells. These cytokines may affect the activity of RAW264.7 cells through autocrine action. DHMBA treatment may inhibit the signal transduction processes mediated by IL-6 and IL-1β produced in RAW264.7 cells.

LPS stimulates osteoclast formation through activation of NF-κB signaling in RAW264.7 cells. LPS treatment enhances TLR4 signaling in macrophages, thereby activating the NF-κB-related signal transduction pathways. It was found that osteoclast formation enhanced by incubation with LPS was suppressed by administration of DHMBA to RAW264.7 cells. This suppression was also observed by DHMBA treatment at the early and late stages of osteoclast formation. The inhibitory effect of DHMBA on osteoclast formation is presumably related to the suppression of NF-κB signaling, which is involved in the reduction of NF-κB p65 level by DHMBA treatment.

### Conclusion

The present invention demonstrates that DHMBA reduces the number of inflammatory macrophages by suppressing the proliferation and promoting cell death of RAW264.7 cells. It was also demonstrated that DHMBA suppressed the production of inflammatory cytokines. Furthermore, DHMBA treatment suppressed osteoclast formation in RAW264.7 cells stimulated with LPS. While it has not yet been elucidated whether DHMBA exhibits anti-inflammatory effects in vivo, the present invention has demonstrated that DHMBA exerts anti-inflammatory effects in vitro in the mouse macrophage RAW264.7 cell model. DHMBA is a functional factor with very low toxicity. DHMBA provides a new strategy in the prevention and treatment of inflammation and is considered pharmacologically important in the treatment of inflammatory conditions.

## Claims

1. An anti-inflammatory agent comprising 3,5-dihydroxy-4-methoxybenzyl alcohol (DHMBA) as an active ingredient, the agent having an anti-inflammatory effect.

2. An inflammatory macrophage activity inhibitor comprising 3,5-dihydroxy-4-methoxybenzyl alcohol (DHMBA) as an active ingredient, the inhibitor having an effect of suppressing activity of an inflammatory macrophage.

3. A RAW264.7 cell proliferation inhibitor, comprising 3,5-dihydroxy-4-methoxybenzyl alcohol (DHMBA) as an active ingredient, the inhibitor having an effect of suppressing proliferation of RAW264.7 cells.

4. A proliferation inhibitor for RAW264.7 cells cultured with LPS, comprising 3,5-dihydroxy-4-methoxybenzyl alcohol (DHMBA) as an active ingredient, the inhibitor having an effect of suppressing proliferation of RAW264.7 cells cultured with LPS.

5. A RAW264.7 cell death promoter, comprising 3,5-dihydroxy-4-methoxybenzyl alcohol (DHMBA) as an active ingredient, the promoter having an effect of promoting death of RAW264.7 cells.

6. An inflammatory cytokine production inhibitor for RAW264.7 cells, comprising 3,5-dihydroxy-4-methoxybenzyl alcohol (DHMBA) as an active ingredient, the inhibitor having an effect of suppressing inflammatory cytokine production in RAW264.7 cells.

7. An inflammatory cytokine production inhibitor for RAW264.7 cells, comprising 3,5-dihydroxy-4-methoxybenzyl alcohol (DHMBA) as an active ingredient, the inhibitor having an effect of suppressing inflammatory cytokine production in RAW264.7 cells.

8. An osteoclast formation inhibitor for RAW264.7 cells, comprising 3,5-dihydroxy-4-methoxybenzyl alcohol (DHMBA) as an active ingredient, the inhibitor having an effect of suppressing osteoclast formation in RAW264.7 cells.
